(19) Europäisches Patentamt
European Patent Office
Office européen des brevets

(11) Publication number: 0 266 006
A2

(12) EUROPEAN PATENT APPLICATION

(21) Application number: 87202052.4

(22) Date of filing: 26.10.87

(51) Int. Cl.[4]: C07K 7/10 , A61K 37/02

(30) Priority: 27.10.86 JP 255312/86

(43) Date of publication of application:
04.05.88 Bulletin 88/18

(84) Designated Contracting States:
AT BE CH DE ES FR GB GR IT LI LU NL SE

(71) Applicant: SHIONOGI SEIYAKU KABUSHIKI KAISHA TRADING UNDER THE NAME OF SHIONOGI & CO. LTD.
12, 3-chome Dosho-machi Higashi-ku
Osaka(JP)

(72) Inventor: Kambayashi, Yoshikazu
3-3-2-1216, Nishimiyahara Yodogawa-ku
Osaka-shi Osaka(JP)
Inventor: Inouye, Ken
131-2-907, Arise Ikawadani-cho Nishi-ku
Kobe-shi Hyogo(JP)

(74) Representative: Bruin, Cornelis Willem et al
OCTROOIBUREAU ARNOLD & SIEDSMA
Sweelinckplein 1
NL-2517 GK The Hague(NL)

(54) **Novel hypotensive diuretic peptides.**

(57) A polypeptide possessing the basic structure of β-hANP but lacking the 1 to 6 and 1′ to 6′ amino acid sequences, *i.e.* Ser-Leu-Arg-Arg-Ser-Ser, and the salts thereof, useful for the treatment of hypertensions, prepared by bimolecular condensation of α-hANP(7-28) at the 7 and 23′ cysteines, and at the 7′ and 23 cysteines, respectively, with disulfide bonds.

Fig. 1

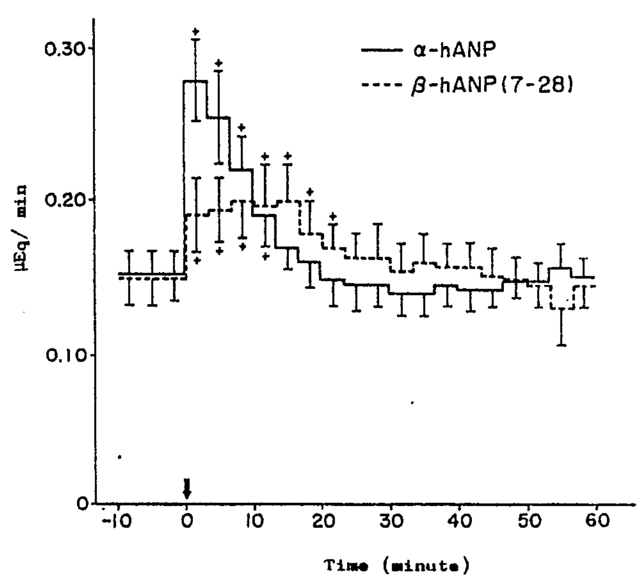


EP 0 266 006 A2

Xerox Copy Centre

## Novel Hypotensive Diuretic Peptides

Background of the Invention

1. Field of the Invention

This invention relates to a novel peptide possessing strong diuretic action, natriuretic action, and hypotensive action, among others.

2. Description of the Prior Art

It is known that a polypeptide possessing strong natriuretic action and hypotensive action (human Atrial Natriuretic Polypeptide = hANP) is present in the atrium of man's heart, and so far three peptides, $\alpha$, $\beta$ and $\gamma$ types, have been isolated and structurally determined.
Of them, an $\alpha$-type of the peptides (hereinafter called $\alpha$-hANP) was first isolated and structurally determined by Kangawa, Matsuo et al., and possessses the following amino acid sequence. (Biochem. Biophys. Res. Commun. 118, 131, 1984).

5 10
H-Ser-Leu-Arg-Arg-Ser-Ser-Cys-Phe-Gly-Gly-Arg-
S – S
15 20
Met-Asp-Arg-Ile-Gly-Ala-Gln-Ser-Gly-Leu-Gly-Cys-
25
Asn-Ser-Phe-Arg-Tyr-OH

The diuretic action of $\alpha$-hANP is said to reach, in a biological study using rats, as much as 1, 500 times that of furosemide which has widely been used as a hypotensive diuretic at the present.

The $\beta$-type peptide ($\beta$-hANP) is a dimer of $\alpha$-hANP, wherein one $\alpha$-hANP in which the cystein S-S coupling at positions 7 and 23 is cleaved is coupled with another molecule of $\alpha$-hANP antiparallel, and is expressed in the following amino acid sequence (Matsuo, Kangawa, Japanese Kokai no. 60-184098; Nature, 313, 397, 1985).

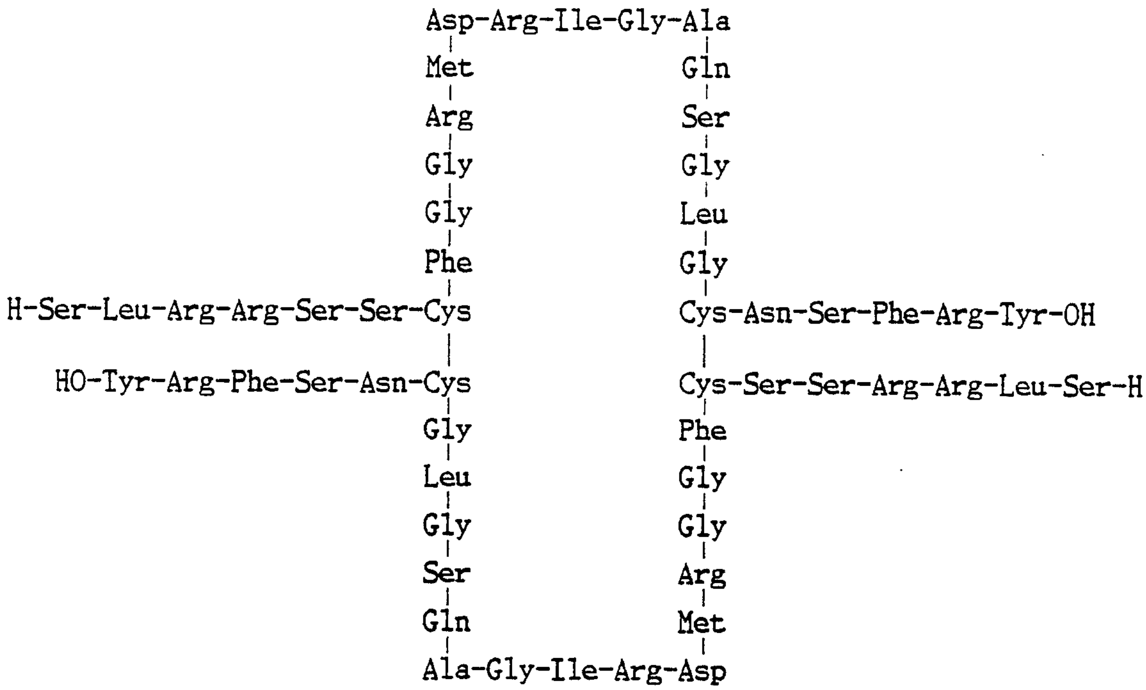

The diuretic action of β-hANP is said to be persistent, unlike that of α type of γ type.

The γ-type peptide (γ-hANP) has an extremely long peptide chain as compared with α type and β type, and is known to be a peptide composed of 126 amino acids (Matsuo, Kangawa, Hayashi, Japanese Kokai no. 60-260596; Nature, 313, 397, 1985).

Besides, as the patent applications relating to atrial peptides, the Japanese Kokai nos. 60-192598 (Atrial peptide), 60-214797 (Novel atrial peptide), 60-262592 (Novel DNA and its application), and 61-7298 (Novel peptide and a medicine using it as an active ingredient) are known among others.

## Summary of the Invention

α-, β-, and γ-hANP are thought to be one of the substances involved in cardiac diseases or essential hypertension. It is an object of the present invention to provide a polypeptide possessing the basic structure of β-hANP but lacking the 1 to 6 and 1′ to 6′ amino acid sequences, *i.e.* Ser-Leu-Arg-Arg-Ser-Ser, and the salts thereof, useful for the treatment of hypertensions, prepared by bimolecular condensation of α-hANP(7-28) at the 7 and 23′ cysteines, and at the 7′ and 23 cysteines, respectively, with disulfide bonds.

## Brief Description of the Drawings

Fig. 1: Transition of urine volume when peptide I and α-hANP as control are administered to rats, in which the thick line indicates peptide I and the dotted line denotes α-hANP. The axis of ordinates represents the urine volume (ml) measured every 3 minutes and 20 seconds per rat, and the axis of abscissas shows the lapse of time in minutes.

Fig. 2: Transition of $Na^+$ excretion volume in the same conditions as in Fig. 1, in which the axis of ordinates denotes the $Na^+$ excretion volume (μ Eq), and the axis of abscissas indicates the lapse of time in minutes.

Description of the Preferred Embodiments

Conventionally, hypertensions have been roughly divided into those with clear causative diseases and others with unclear causative diseases. The former type is classified as secondary hypertension, and it mainly involves the cases due to renal disorder and those caused by endocrine diseases, gestosis, aotarctia and central nervous troubles. On the other hand, the latter type of hypertension with unknown cause is classified as essential hypertension, and about 80 to 90% of hypertensive patients have been classified in this category.

In the case of secondary hypertension, the hypertensive symptoms can be improved by treating the causative diseases, but in the case of essential hypertension with unknown cause, as an expectant treatment, the problem has been solved by administering, for example, hypotensive diuretics or vasodilators. Recently, the above-said atrial peptides are noticed as the substances related to the pathologic causes of heart diseases and essential hypertension, and wide researches are being promoted about the possibility of their application as medicine. The atrial peptides or their analogues isolated or synthesized so far have their problems in the duration of action, stability, and administration, and practical available peptides have not been known yet.

Among the various atrial peptide analogues synthesized for solving the above problems, the peptide expressed in formula (I) below and its salt are known to show a strong hypotensive and diuretic action and excel in the duration of action, and this invention is achieved on the basis of this discovery.

The peptide expressed in formula I basically possesses the structure of β-hANP, but it is characteristic that it lacks the sequences from position 1 to position 6, and from position 1′ to position 6′, that is, the sequence of Ser-Leu-Arg-Arg-Ser-Ser.

```
                         Asp-Arg-Ile-Gly-Ala
                         |                 |
                         Met               Gln
                         |                 |
                         Arg               Ser
                         |                 |
                         Gly               Gly
                         |                 |
                         Gly               Leu
                         |                 |
                         Phe               Gly
                         |                 |
                       H-Cys               Cys-Asn-Ser-Phe-Arg-Tyr-OH
                         |                 |
 HO-Tyr-Arg-Phe-Ser-Asn-Cys               Cys-H
                         |                 |
                         Gly               Phe
                         |                 |
                         Leu               Gly
                         |                 |
                         Gly               Gly
                         |                 |
                         Ser               Arg
                         |                 |
                         Gln               Met
                         |                 |
                         Ala-Gly-Ile-Arg-Asp          ( I )
```

Constituent amino acids in formula I are all of L-type, and their abbreviations are listed below according to the rule of nomenclature by IUPAC (International Union of Pure and Applied Chemistry)-IUB (International Union of Biochemistry).

Ala: Alanine
Arg: Arginine
Asn: Asparagine
Asp: Aspartic acid
Cys: Cysteine
Gln: Glutamine
Gly: Glycine
Ile: Isoleucine
Leu: Leucine

Met: Methionine
Phe: Phenylalanine
Ser: Serine
Tyr: Tyrosine

The polypeptide and its salt expressed in formula I of this invention may be prepared according to the usual method of synthesizing peptide, by the method of synthesis by condensing amino acids one by one and extending the peptide chain, or by the method of coupling fragments composed of two or more amino acids, or by their combined method to prepare two molecules of α-hANP (7-28) chain, in which disulfide bond is formed between the cysteine at position 7 and position 23′ (indicated in the amino acid sequence order of α-hANP) and at position 7′ and position 23.

The present inventors, as a further different method, discovered that it is advantageous to form one of said S-S bonds in an initial stage of synthesis, and the other in a stage after completion of peptide chain and while other protective groups are left over (see the reaction process scheme below).

Condensation of two amino acids, condensation of amino acid and peptide, or condensation of two peptides may be achieved by a conventional condensation method, for example, azide method, mixed acid anhydride method, carbodiimide (dicyclohexylcarbodiimide (DCC), 1-ethyl-3-(3-dimethylaminopropyl) carbodiimide (EDC), etc.) method, active ester method (p-nitrophenyl ester method, N-hydroxyimide succinate (HOSu) ester method, cyanomethylester method, etc.), Woodward reagent K method, carbonyldiimidazole method, oxidation-reduction method, DCC-HOBT (N-hydroxybenzotriazole) method, EDC-HOBt method, etc. These condensation reactions may be conducted either by the solution method or by the solid phase synthetic method. When extending the peptide chain by solid phase method, the C terminal amino acid is bonded with an insoluble carrier. As the insoluble carrier, any substance may be used as far as a detachable bond is formed by reacting with the carboxyl group of C terminal amino acid, and for example, halomethyl resins such as chloromethyl resin and bromomethyl resin, hydroxymethyl resin, aminomethyl resin, benzhydrylamine resin, and t-alkyloxycarboxylhydrazine resin may be used.

As the conventional method of synthesis of peptide, it is necessary to protect/deprotect, as required, the side chain amino acid and carboxyl group at α-position and ω-position of amino acid. Examples of applicable protecting group of amino acid include benzyloxycarbonyl (abbreviated Z), 2-chlorobenzyloxycarbonyl (Z(2-Cl)), 4-nitrobenzyloxycarbonyl (Z($NO_2$)), 4-methoxybenzyloxycarbonyl (Z(OMe)), t-butoxycarbonyl (Boc), t-amyloxylcarbonyl (Aoc), isobornyloxycarbonyl, adamantyloxycarbonyl, 2-(4-biphenyl)-2-propyloxycarbonyl (Bpoc), 9-fluorenylmethoxycarbonyl (Fmoc), methylsulfonylethoxycarbonyl (Msc), trifluoroacetyl, phthalyl, formyl, 2-nitrophenylsulphenyl (NPS), diphenylphosphynothioyl (Ppt), dimethylphosphinothioyl (Mpt).

Examples of protecting group of carboxyl group include benzyl ester (OBzl), 4-nitrobenzyl ester (OBzl-($NO_2$)), t-butyl ester ($OBu^t$), 4-pyridylmethyl ester (OPic), phenacyl ester (OPac), 2, 2, 2-trichloro ester (OTce).

In the process of synthesis of peptide in this invention, it is preferable to protect specific amino acids possessing a functional group other than amino group and carboxyl group in the side chain, such as arginine, cysteine and serine, as required with proper protecting groups. For example, it is preferable to protect the guanidino group of arginine with nitro, p-toluenesulfonyl (Tos), benzyloxycarbonyl (Z), adamantyloxycarbonyl, 4-methoxybenzenesulfonyl, 4-methoxy-2, 6-dimethyl-benzenesulfonyl (Mds), 1, 3, 5-trimethylphenylsulfonyl (Mts), etc., the thiol group of cystein with benzyl, 4-methoxybenzyl (Mob), triphenylmethyl (Tri), acetamidomethyl (Acm), ethylcarbamoyl, 4-methylbenzyl, 2, 4, 6-trimethylbenzyl (Tmb), etc., and the hydroxyl group of serine and tyrosine with benzyl (Bzl), t-butyl, acetyl, tetrahydropyranyl, 2, 6-dichlorbenzyl (Dcb), etc.

Formation of S-S bond between the cysteines at position 7 and position 23′ is achieved by protecting the thiol group of said cysteine with a protecting group different each other at position 7 and position 23, removing one of the protecting groups before forming S-S bond, forming an S-S bond by oxidation, and deprotecting the other thiol group and bonding by oxidation. As a proper thiol protecting group, benzyl (Bzl), 4-methoxybenzyl (Mob), triphenylmethyl (Trt), acetamidomethyl (Acm), ethylaminocarbonyl (Ec), etc, may be used.

An example of preparation of peptide (I) of this invention is practically described according to the reaction scheme formula below.

Reaction scheme

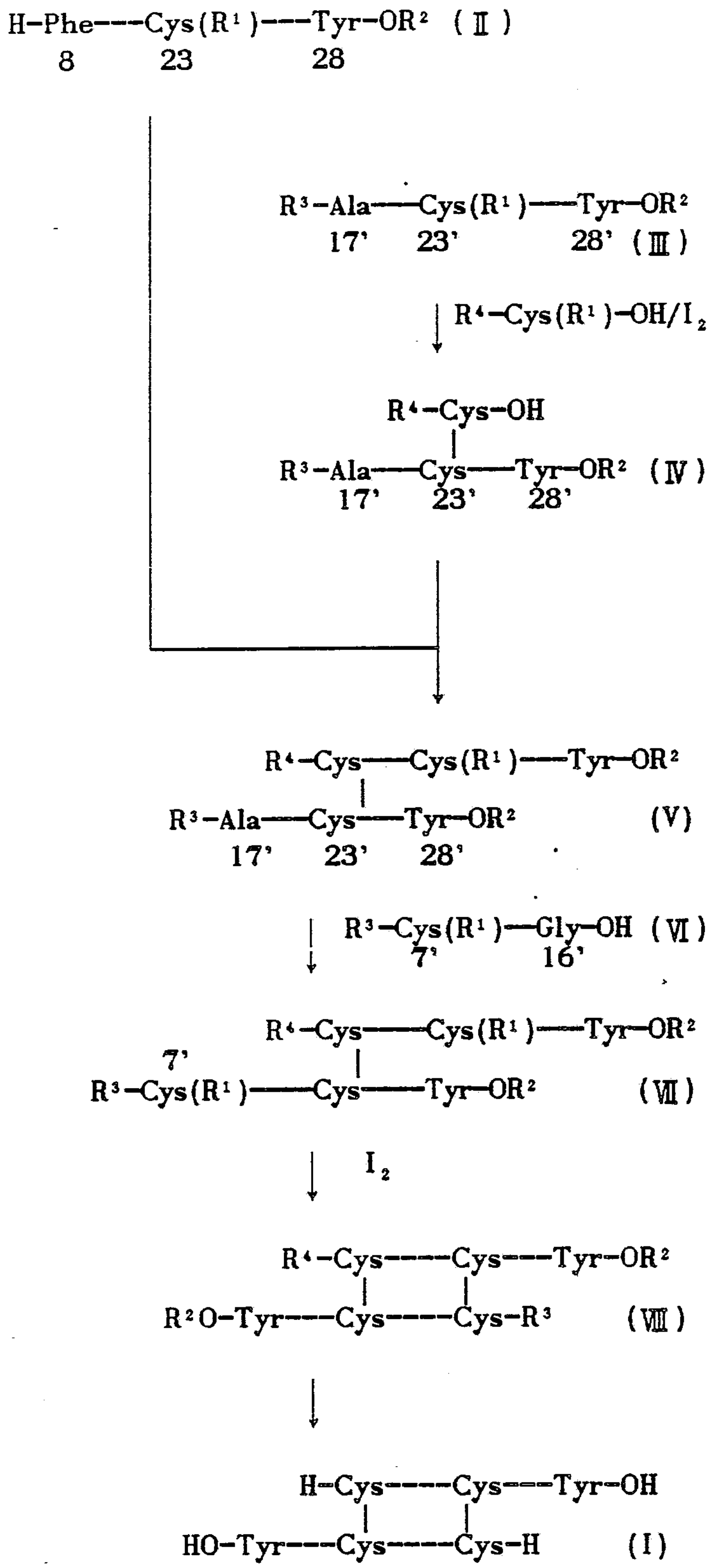


β-hANP-(7-28)

[where $R^1$ denotes a thiol protecting group, $R^2$ is a carboxyl protecting group, and $R^3$ and $R^4$ are amino protecting groups.]

In the above process, preferable examples of thiol protecting group represented by $R^1$ include Acm and Tri. $R^2$ is a protecting group of C terminal amino acid, and Bzl, Pac and Tce are preferable. $R^3$ and $R^4$ are amino protecting groups mutually differing in the elimination condition, and Boc, Aoc, Bpoc, Z, Z(OMe), Fmoc and Msc are preferable.

In the first step, peptide III containing a cysteine residue at position 23 as, for example, Cys (Acm) and possessing a proper chain length is reacted with a large excess amount (for example, 10 equivalent) of the cysteine derivative (for example, Z-Cys(Acm)-OH) in the presence of iodine, and the SH protecting group is eliminated by oxidation, while a disulfide bond is formed to yield peptide IV. What is newly introduced corresponds to the cysteine residue at position 7′.

In the next step, peptide II having phenylalanine as N terminal at position 8′ and cysteine as, for example, Cys (Acm) at position 23′ is condesed with peptide IV obtained above to yield peptide V. The condensation may be carried out in the carbodiimide/additive method, for example, EDC/HOBt method. In the condensation reaction by EDC/HOBt method, N terminal free heneicosapeptide (II) and dodecapeptide (IV) possessing free carboxyl group are dissolved in a solvent used generally in peptide sythesis, for example, dimethyl formamide (DMF), dimethyl sulfoxide (DMSO), hexamethylphosphotriamide (HMPA) or in a mixture of these solvents, and HOBt and EDC are added to allow to react for 1 to 72 hours around 0°C (about -5 to 5°C), or for 1 to 24 hours at room temperature (about 10 to 35°C).
The amino protecting group ($R^3$) of the N terminal amino acid (Ala) of thus obtained polypeptide (V) is selectively removed, and the N-free peptide is condensed with decapeptide (IV) of which C terminal carboxyl protecting group has been preliminarily removed. The condensation reaction in this process is achieved by the condensation reaction with a condensing agent in the presence of additive, for example, by the EDC/HOBt method same as above. The reaction by the EDC/HOBt method may be carried out in the same condition as above.

When the thiol protecting group at position 7′ and 23 of thus obtained peptide (VII) is eliminated by oxidation, a dimer of α-hANP-(7-28) having S-S bonds at positions 7′ and 23, that is, β-hANP (7-28) is obtained. The above two rounds of disulfide bond-forming reaction are easily achieved by mild oxidation reactions, such as iodine treatment. Finally, the remaining protecting group is removed, and the intended peptide (I) is obtained.

In the above process, the elimination may easily be achieved by a mild acid treatment (TFA, HCOOH) if the amino protecting group expressed by $R^3$ is Boc, by catalytic hydrogenation by palladium catalyst if it is a benzyloxycarbonyl protecting group [Z, Z (2Cl), $Z(NO_2)$], or by treatment with piperidine or diethylamine if Fmoc, Msc or the like.

Similarly, the elimination may easily be achieved by catalytic hydrogenation if the carboxyl protecting group expressed by $R^2$ is Bzl, by zinc/acetic acid treatment or catalytic hydrogenation with palladium catalyst if Pac, or by zinc/acetic acid treatment if Tce.

Besides, the protecting group of other functional group held until the final object product may be eliminated at once or stepwise in the final step.

The reaction product and final object in each step may be isolated and refined by conventional separation means for peptide, for example, extraction, recrystallization, chromatography (gel filtration, ion exchange, distribution, adsorption, reverse phase), electrophoresis, or AC distribution.

Incidentally, examples of the salt of peptide expressed in formula I may include inorganic or organic acid-addition salts such as hydrochloride, hydrobromide, nitrate, sulfate, acetate, malate, formate, lactate, tartrate, succinate and citrate, or metal salts such as sodium salt, potassium salt and calcium salt, ammonium salts or amine salts such as ammonium salt and triethyl amine salt.

The starting peptides used in the above process may be prepared in the methods shown in the following working examples and reference examples.

Hereinafter the method of preparing the object compound of this invention is practically described by referring to working examples and reference examples, which are not, however, intended to limit the scope of this invention.

The abbreviations of the protecting groups used in the embodiments and reference examples to follow mean as follows.

Aoc: t-amyloxycarbonyl
Acm: acetamidomethyl
Boc: t-butoxycarbonyl
Bzl: benzyl
Dcb: 2, 6-dichlorobenzyl

Mob: 4-methoxybenzyl
Pac: phenacyl
Tce: 2, 2, 2-trichloroethyl
Tos: tosyl
Z : benzyloxycarbonyl

Example

Preparation of human β-ANP-(7-28)

(1) Z-Cys(Acm)-OH dicyclohexylamine (DCHA) salt (1)

Boc-Cys(Acm)-OH (8.77 g, 30 mmol) is dissolved in trifluoroacetic acid (TFA) (20 ml) and is treated for 40 minutes in ice chilling. The residue obtained by distilling off TFA under reduced pressure is dissolved and neutralized with aqueous solution of IM-sodium hydrogencarbonate, to which aqueous solution of IM-sodium hydroxide (60 ml) is added. Carbobenzoxychloride (Z-CL, 7.68 g, 45 mmol) is added to the above mixture and the resulting mixture is stirred for 2 hours in ice chilling and another 3 hours at 23°C. The reaction mixture is washed with ether, and adjusted to pH 2 with concentrated hydrochloric acid. The precipitating oily material is extracted with ethyl acetate, and the washing is repeated with saturated sodium chloride aqueous solution (hereinafter referred to as saturated brine) until the mixture become pH 5, and dried on magnesium sulfate. The filtrate is evaporated under reduced pressure, and DCHA (5.85 ml, 30 mmol) is added to the residue. The precipitated crystals are collected by filtration and recrystallized from methanol-ethyl acetate to give 10.8 g of the captioned compound (yield 71%); mp 159 -161°C.

(2) Boc-Ala-Gln-Ser(Bzl)-Gly-Leu-Gly-Cys(Z-Cys-OH)-Asn-Ser(Bzl)-Phe-Arg(Tos)-Tyr(Dcb)-OBzl ( 2)

Compound 1 (2.03 g, 4.0 mmol) is dissolved in 50% ethanol (10 ml), and is shaken for 30 minutes at 23°C, together with strongly acidic cation-exchange resin Dowex 50Wx8 ($H^+$ type, 20 ml). The mixture is filtrated and the filtrate is evaporated to dryness under reduced pressure to give Z-Cys(Acm)-OH, which is dissolved with Boc-Ala-Gln-Ser(Bzl)-Gly-Leu-Gly-Cys(Acm)-Asn-Ser(Bzl)-Phe-Arg(Tos)-Tyr(Dcb)-OBzl (III: $R^1$ = Acm; $R^2$ = Bzl; $R^3$ = Boc) (822 mg, 0.4 mmol) in N,N-dimethylformamide (DMF)(30 ml). Iodine (3.05 g) is added thereto and the mixture is allowed to react at 23 °C for 30 minutes. After the excess amount of iodine is neutralized with 1M-sodium thiosulfate aqueous solution, the solvent is evaporated under reduced pressure. Ethyl acetate and water are added to the residue and the resulting precipitate is collected by filtration. This is reprecipitated from DMF-methanol to yield 620 mg of the captioned compound (yield 69%); mp 249-250 °C (decomposed).

(3) Boc-Phe-Gly-Gly-OH•DCHA salt (3)

Boc-Phe-Gly-Gly-OBzl (24) (2.50 g, 5.3 mmol) is dissolved in ethanol (10 ml), and catalytic hydrogenolysis is performed at ordinary pressure in the presence of palladium-black. After elapse of 6 hours, the catalyst is filtered off, and the filtrate is evaporated at reduced pressure. The residue is dissolved in ethyl acetate and DCHA (1.03 ml, 5.3 mmol) is added thereto, and the precipitated crystals are collected by filtration to yield 2.89 g of the captioned compound (yield 97%); mp 155 -158°C.

(4) Boc-Phe-Gly-Gly-Arg(Tos)-Met-Asp(OBzl)-Arg(Tos)-Ile-Gly-OTce (4)

Aoc-Arg(Tos)-Met-Asp(OBzl)-Arg(Tos)-Ile-Gly-OTce (30 ) (836 mg, 0.6 mmol) is dissolved in TFA (5 ml) in ice chilling. After elapse of 40 minutes, TFA is distilled away under reduced pressue and ether is added to the residue. The resulting precipitate is collected by the filtration to give H-Arg(Tos)-Met-Asp-(OBzl)-Arg-(Tos)-Ile-Gly-OTce-TFA salt, which is dissolved in DMF (10 ml) together with Boc-Phe-Gly-Gly-OH prepared by (336 mg, 0 .6 mmol) with Dowex 50Wx8 according to the method specified in (2). In the presence of 1-hydroxybenzotriazole (HOBt) (162 mg, 1.2 mmol), 1-ethyl-3-(3-dimethylaminopropyl)-carbodiimide (EDC) (279 mg, 1.8 mmol) is added to the above mixture and the resulting mixture is allowed to react for 18 hours at 0°C. The solvent is distilled away and ethyl acetate is added to the rèsidue, and the resulting precipitate is reprecipitated from DMF-methanol-ethyl acetate to yield 756 mg of the captioned compound (yield 77%); mp 218 -219°C (decomposed).

(5) Boc-Phe-Gly-Gly-Arg(Tos)-Met-Asp(OBzl)-Arg(Tos)-Ile-Gly-OH (5)

Compound 4 (500 mg, 0.3 mmol) is dissolved in DMF (2 ml), and acetic acid (2 ml) is added. To this mixture is added zinc powder (500 mg) and the resulting mixture is allowed to react for 90 minutes at 23°C. The insoluble inorganic material is filtrated off and the filtrate is distilled under reduced pressure. The residue is washed with 0.1M hydrochloric acid and is reprecipitated from DMF-methanol-ethyl acetate to yield 397 mg of the captioned compound (yield 86%); mp 202 - 204°C (degenerated at 184°C).

(6) Boc-Phe-Gly-Gly-Arg(Tos)-Met-Asp(OBzl)-Arg(Tos)-Ile-Gly-Ala-Gln-Ser(Bzl)-Gly-Leu-Glr-Cys(Acm)-Asn-Ser(Bzl)-Arg(Tos)-Tyr(Dcb)-OBzl (6)

Boc-Ala-Gln-Ser(Bzl)-Gly-Leu-Gly-Cys(Acm)-Asn-Ser(Bzl)-Phe-Arg(Tos)-Tyr(Dcb)-OBzl (453 mg, 0.22 mmol) is treated with TFA in the same manner as in (4) above mentioned to give H-Ala-Gln-Ser(Bzl)-Gly-Leu-Gly-Cys(Acm)-Asn-Ser(Bzl)-Phe-Arg(Tos)-Tyr(Dcb)-OBzl • TFA salt, which is dissolved in DMF (5 ml) together with compound 5 (331 mg, 0.22 mmol) and HOBt (89 mg, 0.66 mmol). EDC (102 mg, 0.66 mmol) is added thereto in ice chilling. The mixture is allowed to react for 18 hours at 0°C, methanol is added. The resulting precipitate is collected by filtration to yield 655 mg of the captioned compound (yield 86%); mp 248 -250°C (decomposed).

(7)

Z-Cys-Phe-Gly-Gly-Arg(Tos)-Met-Asp(OBzl)
Ser(Bzl)-Gln-Ala-Gly-Ile-Arg(Tos)
Gly-Leu-Gly-Cys(Acm)-Asn-Ser(Bzl)
Bzl-O-Tyr(Dcb)-Arg(Tos)-Phe

Boc-Ala-Gln-Ser(Bzl)-Gly-Leu-Gly-Cys-Asn
Bzl-O-Tyr(Dcb)-Arg(Tos)-Phe-Ser(Bzl)

(7)

Compound 6 (310 mg, 0.09 mmol) is treated with TFA in the same manner as in (4) to prepare H-Phe-Gly-Gly-Arg(Tos)-Met-Asp(OBzl)-Arg(Tos)-Ile-Gly-Ala-Gln-Ser(Bzl)-Gly-Leu-Gly-Cys(Acm)-Asn-Ser(Bzl)-Phe-Arg(Tos)-Tyr(Dcb)-OBzl • TFA salt. This is dissolved in a mixed solvent of dimethyl sulfoxide (DMSO) (2 ml) and DMF (4 ml), together with compound 2 (202 mg, 0.09 mmol), and EDC (140 mg, 0.9 mmol) is added thereto. The mixture is allowed to react for 64 hours in ice chilling in the presence of HOBt (122 mg, 0.9 mmol). Methanol is added and resulting precipitate is collected by filtration to yield 484 mg of the captioned compound (yield 97%); mp 249 -252°C (decomposed).

(8)

Z-Cys-Phe-Gly-Gly-Arg(Tos)-Met-Asp(OBzl)-
Ser(Bzl)-Gln-Ala-Gly-Ile-Arg(Tos)-
Gly-Leu-Gly-Cys(Acm)-Asn-Ser(Bzl)-
Bzl-O-Tyr(Dcb)-Arg(Tos)-Phe

Boc-Cys(Acm)-Phe-Gly-Gly-Arg(Tos)-Met
Gly-Ile-Arg(Tos)-Asp(OBzl)
Ala-Gln-Ser(Bzl)-Gly-Leu-Gly-Cys-
Bzl-O-Tyr(Dcb)-Arg(Tos)-Phe-Ser(Bzl)-Asn

(8)

Compound 7 (445 mg, 0.08 mmol) is treated with TFA in the same manner as in (4) to prepare corresponding TFA salt, which is dissolved in DMSO (2 ml)-DMF (4 ml) together with Boc-Cys(Acm)-Phe-Gly-Gly-Arg(Tos)-Met-Asp(OBzl)-Arg(Tos)-Ile-Gly-OH (134 mg, 0.08 mmol). HOBt (108 mg, 0.8 mmol) and EDC (124 mg, 0.8 mmol) are added thereto in ice chilling, and the resulting mixture is allowed to react for 18 hours. To the reaction mixture, methanol is added and the resulting precipitate is collected by filtration to yield 534 mg of the captioned compound (yield 94%); mp 252 -254°C (decomposed).

(9) Human β-ANP-(7-28)

Compound 8 (100 mg, 14 μmol) is dissolved in a mixed solvent of DMSO (2 ml)-DMF (5 ml). A solution of iodine (73 mg) in DMF (7 ml) is added thereto and the mixture is allowed to react for 75 minutes at 23°C. Added 1M sodium thiosulfate, the reaction mixture is evaporated under reduced pressure, and water is added. The resulting precipitate is collected by filtration, washed with water, ethanol and ethyl ether successively, and dried. After the resulting solution is treated with TFA in the same manner as in (4), the mixture is treated with HF (5 ml) for 60 minutes at 0°C in the presence of anisole (0.4 ml)-dimethylsulfide (0.2 ml). HF is distilled away under reduced pressure and 0.2M acetic acid and ethyl ether are added to the residue, and the mixture is shaken well. After neutralizing the aqueous layer with concentrated ammonia water, water is distilled away under reduced pressure together with n-butyl alcohol. The residue is sequentially purified through reverse phase column (Wako RQ-2, 24 mm IDx360 mm, 0.1% TFA containing 10 -60% acetonitrile) and reverse phase HPLC (YMC Pak A-342, 20 mm IDx150 mm, 25% acetonitrile containing 0.1% TFA), and the eluate is freeze-dried to yield 4.3 mg of the captioned compound.
Amino acid analytical values (in 6M hydrochloric acid containing 5% thioglycollic acid, hydrolysis at 110°C, 24 hours):
Asp 3.97(4), Ser 3.55(4), Glu 2.15(2) Gly 9.87(10), Ala 2.16(2), Met 2.05(2) Ile 1.98(2), Leu 2.06(2), Tyr 2.03-(2) Phe 4.00(4), Arg 5.99(6).

Preparation of the starting peptide

1. Preparation of Boc-Ala-Gln-Ser(Bzl)-Gly-Leu-Gly-Cys(Acm)-Asn-Ser(Bzl)-Phe-Arg(Tos)-Tyr(Dcb)-OBzl (III:$R^1$ = Acm; $R^2$ = Bzl; $R^3$ = Boc)

(1) Bos-Tyr(Dcb)-OBzl (9)

Boc-Tyr(Dcb)-OH (22 g, 50 mmol) is dissolved in 100 ml of methylene chloride, and dicyclohexylcarbodiimide (DDC; 11.3 g, 55 mmol) is added with stirring in ice chilling. To this mixture, benzylalcohol (10.4 ml, 100 mmol) and 4-dimethylaminopyridine (DMAP; 6.1 g, 50 mmol) are added and the resulting mixture is allowed to stand for 28 hours at 0°C. The insoluble material is filtered off and the filtrate is evaporated under reduced pressure. The residue is dissolved in ethyl acetate, washed with 1M hydrochloric acid and saturated sodium hydrogencarbonate aqueous solution (hereinafter called sodium bicarbonate solution) successively, dried on magnesium sulfate, and filtrated. The filtrate is distilled under reduced pressure and the residue is recrystallized from ethyl acetate-petroleum ether to yield 23.4 g of the captioned compound (yield 88%);
$[\alpha]_D$ -10.2 ± 0.5° (23.5 °C, c 1.0, methanol).

(2) Aoc-Arg(Tos)-Tyr(Dcb)-OBzl (10)

Compound 9 (15.9 g, 30 mmol) is dissolved in trifluoroacetic acid (TFA)-methylene chloride (1:1, 40 ml), and the mixture is allowed to stand for 30 minutes at 0°C. The reaction mixture is evaporated under reduced pressure and the residue treated with 50% potassium carbonate aqueous solution in the presence of ethyl acetate, and the organic layer is dried on magnesium sulfate. The filtrate is evaporated under reduced pressure and the residue is dissolved in methylene chloride. The mixture is added to the methylene chloride solution of the acid anhydride prepared from the Aoc-Arg(Tos)-OH (15.83 g, 30 mmol) and DCC (6.20 g, 30 mmol) according to the conventional method in ice chilling and the resulting mixture is allowed to react for 18 hours. After removing the insoluble material, the reaction mixture is evaporated under reduced pressure. The residue is dissolved in ethyl acetate, washed with ice-chilled 1M hydrochloric acid and saturated sodium bicarbonate solution, and dried on magnesium sulfate. The filtrate is evaporated under reduced pressure and the resulting oil is refined with silica gel column chromatography (250 g, 97% chloroform-3% methanol) to give 20.2 g of the captioned compound (yield 79%).
$[\alpha]_D$ -8.5 ± 0.5° (23.5 °C, c 1.0, methanol).

(3) Boc-Phe-Arg(Tos)-Tyr(Dcb)-OBzl (11)

Compound 10 (20 g, 23.4 mmol) is treated with TFA in the same manner as in (2) to prepare H-Arg-(Tos)-Tyr(Dcb)-OBzl, which is dissolved in dimethylformamide (DMF), and Boc-Phe-OSu (8.5 g, 23.4 mmol) is added thereto. The mixture is allowed to react for 18 hours at 23°C. DMF is distilled off under reduced pressure, and the residue is dissolved in ethyl acetate, washed with water and dried on magnesium sulfate. The filtrate is evaporated under reduced pressure and the resulting jelly-like material is refined with silica gel column chromatography (300 g, 98% chloroform-2% methanol) to yield 20.6 g of the captioned compound (yield 89%).
$[\alpha]_D$ -10.4 ± 0.5° (24 °C, c 1.0, methanol).

(4) Boc-Ser(Bzl)-Phe-Arg(Tos)-Tyr(Dcb)-OBzl (12)

Compound 11 (13.2 g, 13.4 mmol) is dissolved in TFA-methylene chloride (1:1), and allowed to react for 30 minutes in ice chilling. After the reaction mixture is distilled under reduced pressure, ether is added to the residue. The resulting precipitate is collected by filtration to give H-Phe-Arg(Tos)-Tyr(Dcb)-OBzl TFA salt, which is dissolved in DMF (100 ml). tri-n-butylamine (3.5 ml, 14.6 mmol) and Boc-Ser(Bzl)-OSu (5.3 g, 13.4 mmol) are added thereto, and the mixture is allowed to stand for 18 hours at 23°C. The DMF is distilled off under reduced pressure and the residue is treated in the same manner as in (1). The residue is reprecipitated from ethyl acetate-ethyl ether to yield 13.9 g of the captioned compound.
$[\alpha]_D$ -14.4 ± 0.5° (24 °C, c 1.0, methanol).

(5) Boc-Asn-Ser(Bzl)-Phe-Arg(Tos)-Tyr(Dcb)-OBzl (13)

Compound 12 (11.7 g, 10 mmol) is treated with TFA in the same manner as in (4) to prepare H-Ser-(Bzl)-Phe-Arg(Tos)-Tyr(Dcb)-OBzl • TFA salt, which is dissolved in DMF and the mixture is chilled in ice. To this, tri-n-butylamine (4.0 ml, 17 mmol) and Boc-Asn-ONp (3.53 g, 10 mmol) are added, and the resulting mixture is allowed to react for 18 hours at 23 °C. Furthermore, 1 g of active ester is added, and the mixture is allowed to stand for 18 hours. Distilling off the DMF at reduced pressure, ethyl acetate is added to the residue, and the resulting precipitate is reprecipitated from chloroform-methanol-ethylether to give 11.3 g of the captioned compound (yield 88%).
$[\alpha]_D$ -24.4 ± 0.6° (25 °C, c 1.0, methanol).

(6) Boc-Cys(Acm)-Asn-Ser(Bzl)-Phe-Arg(Tos)-Tyr(Dcb)-OBzl (14)

Compound 13 (1.5 g, 1.17 mmol) is treated with TFA in the same manner as in (4) to prepare H-Asn-Ser(Bzl)-Phe-Arg(Tos)-Tyr(Dcb)-OBzl • TFA salt, which is dissolved in DMF. Tri-n-butylamine (0.28 ml, 1.17 mmol) and Boc-Cys-(Acm)-OSu (0.525 g, 1.35 mmol) are added thereto at 0°C, and the mixture is allowed to react for 18 hours at 23°C. DMF is evaporated under reduced pressure and ethyl acetate is added to the residue. The resulting precipitate is collected by filtration and reprecipitated from chloroform-methanol-ethyl acetate to yield 1.44 g of the captioned compound (yield 85%); mp 184 -6°C.
$[\alpha]_D$ -19.9 ± 0.6° (23 °C, c 1.0, DMF).

(7) Boc-Gly-OPac (15 )

Boc-Gly-OH (25 g, 143 mmol) is suspended in water, and cesium carbonate (23.2 g, 143 mmol) is added in ice chilling. Water is distilled off under reduced pressure and the resulting crystalline residue is dried under reduced pressure over diphosphorus pentaoxide. This is dissolved in DMF and phenacyl bromide (28.4 g, 143 mmol) is added. The mixture is allowed to react for 18 hours at 23°C. The insoluble material is removed by filtration and the filtrate is evaporated under reduced pressure. The residue is dissolved in ethyl acetate, washed with water and saturated sodium bicarbonate solution successively, and dried on magnesium sulfate. The filtrate is evaporated to dryness under reduced pressure. The residue is crystallized and recrystallized from ethylether-petroleum ether to yield 36.9 g of the captioned compound (yield 88%); mp 61.5 - 62°C.

(8) Boc-Leu-Gly-OPac ( 16)

Compound 15 (9.68 g, 33 mmol) is dissolved in methylene chloride and a same volume of trifluoroacetic acid (TFA) is added, and the mixture is allowed to stand for 30 minutes at 23°C. The solvent is evaporated under reduced pressure and ethyl ether is added to the residue. The resulting precipitate is collected by filtration and dissolved in DMF. Boc-Leu-OSu (9.85 g, 30 mmol), tri-n-butylamine (7.8 ml, 33 mmol) and 1-hydroxybenzotriazole (HOBt; 4.05 g, 30 mmol) are added thereto in ice chilling, and the mixture is allowed to stand for 18 hours at 0°C. DMF is distilled away and the residue is dissolved in ethyl acetate, washed successively with 1 M hydrochloric acid and saturated sodium bicarbonate solution, and dried on magnesium sulfate. The filtrate is decolored with aluminium oxide and recrystallized from ethyl acetate-petroleum ether to yield 8.54 g of the captioned compound (yield 70%); mp 111 -113°C.

(9) Boc-Ser(Bzl)-Gly-OPac (17)

Compound 15 (11.8 g, 40 mmol) is treated in the same as in (8), and to yield 13.6 g of the captioned compound (yield 72 %).
$[\alpha]_D$ -0.7 ± 0.4° (23.5 °C, c 1.0, methanol).

(10) Boc-Gln-Ser(Bzl)-Gly-OPac (18)

Compound 17 (13.5 g, 28.7 mmol) is treated with TFA in the same manner as in (8) to prepare H-Ser-(Bzl)-Gly-OPac • TFA salt, which is dissolved in DMF (60 ml). Tri-n-butylamine (16 ml, 67.2 mmol), Boc-Gln-ONp (10.5 g, 28.7 mmol) and HOBt (3.88 g, 67.2 mmol) are added to the above solution and the mixture is allowed to react for 20 hours at 23°C. DMF is distilled away under reduced pressure, methanol is added to the residue, and the resulting crystals are collected by filtration and recrystallized from aqueous methanol to yield 14.8 g of the captioned compound (yield 86%).
$[\alpha]_D$ -11.7 ± 0.5° (23.5 °C, c 1.0, methanol).

(11) Boc-Ala-Gln-Ser(Bzl)-Gly-OPac (19)

Compound 18 (13.8 g, 23 mmol) is treated with TFA in the same manner as in (8) to prepare H-Gln-Ser(Bzl)-Gly-OPac TFA salt, which is dissolved in DMF (100 ml) and tri-n-butylamine (7.0 ml, 29.4 mmol) and Boc-Ala-OSu (7.3 g, 25.4 mmol) are added. The mixture is allowed to react for 18 hours at 23°C. DMF is distilled away under reduced pressure and the residue is recrystallized from methanol-ethyl acetate-water to give 13.1 g of the captioned compound (yield 85%);
$[\alpha]_D$ -6.5 ± 0.5° (23.5 °C, c 1.0, DMF).

(12) Boc-Ala-Gln-Ser(Bzl)-Gly-OH (20)

Compound 19 (12.2 g, 18.2 mmol) is dissolved in DMF (30 ml), and 85% acetic acid (30 ml) is added. To this, activated zinc powder (12.6 g, 193 mmol) is added. The mixture is allowed to react for 4 hours at 23°C. The insoluble material is filtrated off, then the solvent is distilled away under reduced pressure. The residue is dissolved in ethyl acetate, washed successively with 1 M hydrochloric acid and water. After dried on magnesium sulfate, the filtrate is evaporated under reduced pressure, and the residue is recrystallized from methanol-ethylether to give 7.95 g of the captioned compound (yield 76%).
$[\alpha]_D$ -26.2 ± 0.7° (24 °C, c 1.0, methanol).

(13) Boc-Ala-Gln-Ser(Bzl)-Gly-Leu-Gly-OPac (21)

Compound 20 (4.0 g, 7 mmol) is dissolved in DMF (15 ml) and N-hydroxysuccinimide (HOSu; 1.0 g, 8.4 mmol) and dicyclohexylcarbodiimide (DCC; 1.73 g, 8.4 mmol) are added in ice chilling. The mixture is allowed to react for 6 hours at 0 °C, and the insoluble matertial is filtered off to prepare the corresponding active ester solution. On the other hand, compound 16 (2.9 g, 7 mmol) is treated with TFA in the same manner as in (8) to prepare H-Leu-Gly-OPac • TFA salt, which is dissolved in DMF (30 ml). The active ester solution prepared above is added thereto. Furthermore, tri-n-butylamine (6 ml, 25 mmol) and HOBt (0.95 g, 7.0 mmol) are added, and the mixture is allowed to react for 72 hours at 0°C. Ethyl ether is added to the reaction mixture and the resulting precipitate is collected by filtration, and recrystallized from methanol-ethyl acetate-water to yield 4.97 g of the captioned compound (21) (yield 85%).
$[\alpha]_D$ -32.5 ± 0.7° (23 °C, c 1.0, methanol).

(14) Boc-Ala-Gln-Ser(Bzl)-Gly-Leu-Gly-OH (22)

Boc-Ala-Gln-Ser(Bzl)-Gly-Leu-Gly-OPac (21) (2.72 g, 3.2 mmol) is dissolved in 85% acetic acid aqueous solution, and zinc powder (2.1 g, 32 mmol) is added, and the mixture is stirred for 2 hours at 23°C. The insoluble material is filtrated off, the filtrate is evaporated under reduced pressure. The resulting residue is washed with 1M hydrochloric acid and recrystallized from aqueous methanol to yield 1.87 g of the captioned compound (22) (yield 80%).
Anal. Calcd. (%) for $C_{33}H_{51}N_7O_{11}$• 1/2 $H_2O$ : C54.24; H7.17; N13.42
Found (%): C54.23; H6.99; N13.39

(15) Boc-Ala-Gln-Ser(Bzl)-Gly-Leu-Gly-Cys(Acm)-Asn-Ser(Bzl)-Phe-Arg(Tos)-Tyr(Dcb)-OBzl (III:$R^1$ = Acm; $R^2$ = Bzl; $R^3$ = Boc)

Compound 14 (1.30 g, 0.89 mmol) is treated with TFA in the same manner as in (4), and the obtained corresponding TFA salt is dissolved in DMF (10 ml) together with Boc-Ala-Gln-Ser(Bzl)-Gly-Leu-Gly-OH (22) (650 mg, 0.89 mmol) and 1-hydrobenzotriazole (180 mg, 1.3 mmol). To this, in ice chilling, 1-ethyl-3-(3-dimethylaminopropyl)-carbodiimide (EDC) (210 mg, 1.3 mmol) is added, and the mixture is allowed to stand at 0°C for 4 hours, and at 25°C for 16 hours. DMF is distilled off and methanol is added thereto. The resulting percipitate is repercipitated from DMF-methanol to yield 1.8 g of the captioned compound (III) (yield 96%); mp 240 -242°C.

2. Preparation of Boc-Phe-Gly-Gly-OBzl (24)

(1) Boc-Phe-Gly-OBzl ( 23)

Glycine benzyl ester p-toluene sulfonate (10, 1 g, 30 mmol) is dissolved in DMF (50 ml), and tri-n-butylamine (7.8 ml, 33 mmol) and Boc-Phe-OSu (10.9 g, 30 mmol) are added. The mixture is allowed to react for 18 hours at 23°C. DMF is distilled away under reduced pressure and the residue is dissolved in ethyl acetate and washed successively with cold 1 M-hydrochloric acid and saturated sodium bicarbonate solution, and the organic layer is dried on magnesium sulfate. After evaporating the filtrate under reduced pressure, petroleum ether is added, and resulting crystals are recrystallized from ethyl acetate-petroleum ether to yield 7.8 g of the captioned compound (yield 63 %); mp 135.5-136.5 °C.

(2) Boc-Phe-Gly-Gly-OBzl (24)

Compound 23 (7.8 g, 18.9 mmol) is suspended in ethanol, and catalytic hydrogenolysis is carried out at ordinary pressure in the presence of palladium-black. The catalyst is filtrated off and the filtrate is distilled under reduced pressure. The residue is dissolved in methylene chloride, and a solution of H-Gly-OBzl, which is prepared from treating glycine benzyl ester p-toluene sulfonate (7.65 g, 23 mmol) in. 50 % potassium carbonate aqueous solution in methylene chloride is added thereto. In ice chilling, DCC (3.9 g, 18.9 mmol) is added and the resulting mixture is allowed to react for 18 hours. Concentrating the filtrate, the residue is recrystallized from ethyl acetate-petroleum ether to give 8.6 g of the captioned compound (yield 97%); mp 113 - 114°C.

3. Preparation of Aoc-Arg(Tos)-Met-Asp(OBzl)-Arg(Tos)-Ile-Gly-OTce (30)

(1) Boc-Gly-OTce (25)

Boc-Gly-OH (52.6 g, 0.3 mol) is dissolved in methylene chloride (300 ml), and 2, 2, 2-trichloroethanol (44.9 g, 0.3 mol) and 4-dimethylaminopyridine (DMAP; 3.67 g, 0.03 mol) are added and chilled with ice. Dicyclohexylcarbodiimide (DCC; 61.9 g, 0.3 mol) is added by several divided portions to react for 18 hours at 4°C. Removing the insoluble material, the filtrate is evaporated under reduced pressure. The residue is dissolved in ethyl acetate (400 ml) and successively washed with 1M citric acid aqueous solution, saturated sodium chloride aqueous solution, and saturated sodium carbonate aqueous solution (hereinafter called saturated sodium bicarbonate solution), and dried on magnesium sulfate. The oil obtained by evaporating the filtrate under reduced pressure is dissolved in petroleum ether and is allowed to stand at 4°C. The resulting crystals are collected by filtration to yield 79 g of the captioned compound (yield 86%); mp 73.5-74.0 °C.

(2) Boc-Ile-Gly-OTce (26 )

Compound 25 (20.2 g, 66 mmol) is dissolved in trifluoroacetic acid (TFA) (30 ml), and is allowed to stand at 0°C for 30 minutes. The residue obtained by distilling away the TFA under reduced pressure is dissolved in methylene chloride and shaken together with 50% potassium carbonate aqueous solution. The organic layer is separated, and dried on magnesium sulfate. The filtrate is concentrated to about 150 ml under reduced pressure, and Boc-Ile-OH • 1/2 $H_2O$ (15.1 g, 63 mmol) is added, and DCC (13.0 g, 63 mmol) is added by several divided portions in ice chilling. The mixture is allowed to react for 1 hour in ice chilling and 5 hours at 23°C. The insoluble material is filtrated off and the filtrate is evaporated under reduced pressure. The residue is dissolved in ethyl acetate, and successively washed with cold 1M-hydrochloric acid and saturated sodium bicarbonate solution, and dried on magnesium sulfate. The oil obtained by evaporating the filtrate under reduced pressure is dissolved in petroleum ether, and is allowed to stand at 4°C. The precipitating crystals are collected by filtration to yield 13.4 g of the captioned compound (yield 48 %); mp 92 - 4 °C.

(3) Aoc-Arg(Tos)-Ile-Gly-OTce (27)

Compound 26 (8.39 g, 20 mmol) is treated with TFA in the same manner as in (2) to prepare methylene chloride solution (about 100 ml) of H-Ile-Gly-OTce, to which methylene chloride solution (50 ml) of Aoc-Arg-(Tos)-OH • 1/4 • AcOEt • 1/2 $H_2O$ (9.47 g, 20 mmol) is added. In ice chilling, DCC (4.13 g, 20 mmol) is added and the mixture is allowed to react for 1 hours at 4 °C and 6 hours at 23 °C. The reaction mixture is filtrated and evaporated under reduced pressure. The residue is dissolved in ethyl acetate, and washed successively with cold 1 M hydrochloric acid and saturated sodium bicarbonate solution, and dried on magnesium sulfate. After evaporating the filtrate under reduced pressure, the residue is dissolved in chloroform, and refined by silica gel column chromatography (300 g, 98% chloroform-2% methanol) to yield 12.6 g of the captioned compound (yield 80 %).

Anal. Calcd. (%) for $C_{29}H_{45}N_6O_8SCl_3$ : C46.81; H6.10; N11.29; S4.31; C114.29

Found (%): C46.18; H6.04; N11.03; S4.12; C114.90

(4) Boc-Asp(OBzl)-Arg(Tos)-Ile-Gly-OTce (28)

Compound 27 (11.2 g, 15.1 mmol) is dissolved in TFA (30 ml), and is allowed to stand for 30 minutes at 4°C. Ethyl ether is added to the residue obtained by distilling off the TFA at reduced pressure. The resulting precipitate is collected by filtration to give H-Arg(Tos)-Ile-Gly-OTce • TFA salt, which is dissolved in dimethylformamide (DMF) (50 ml), and Boc-Asp(OBzl)-OSu (6.33 g, 15.1 mmol) and tri-n-butylamine (4.35 ml, 18.3 mmol) are added. The mixture is allowed to react for 5 hours at 23 °C and the reaction mixture is distilled under reduced pressure. The residue is dissolved in ethyl acetate, washed successively with cold 1M hydrochloric acid and saturated sodium bicarbonate solution and dried on magnesium sulfate. The filtrate is evaporated under reduced pressure, and petroleum ether is added. The resulting precipitate is collected by filtration and reprecipitated from ethyl acetate-petroleum ether to yield 13.1 g of the captioned compound (yield 93%); 110 -113°C.

(5) Boc-Met-Asp(OBzl)-Arg(Tos)-Ile-Gly-OTce ( 29)

Compound 28 (2.98 g, 3.19 mmol) is dissolved in chloroform-TFA (1:4) (20 ml), and is allowed to stand for 40 minutes at 4°C. The solvent is distilled away under reduced pressure and the residue is dissolved in chloroform, washed with saturated sodium bicarbonate solution and dried on magnesium sulfate. The residue obtained by evaporating the filtrate under reduced pressure is dissolved in DMF (20 ml), and Boc-Met-OSu (1.22 g, 3.51 mmol) is added. The mixture is allowed to react for 18 hours at 23 °C. By distilling away the DMF under reduced pressure, the residue is dissolved in chloroform, washed with water and dried on magnesium sulfate. After concentrating the filtrate, ether is added and the resulting percipitate is collected by filtration to yield 3.28 g of the captioned compound (yield 95 %); mp 147 - 155 °C.

(6) Aoc-Arg(Tos)-Met-Asp(OBzl)-Arg(Tos)-Ile-Gly-OTce (30)

Compound 29 (3.05 g, 2.86 mmol) is treated with TFA in the same manner as in (5) to prepare H-Met-Asp(OBzl)-Arg(Tos)-Ile-Gly-OTce • TFA salt, which is dissolved in DMF (20 ml), and Aoc-Arg(Tos)-OH • 1/4 AcOEt • 1/2 $H_2O$ (1.35 g, 2.86 mmol) is added, and furthermore 1-ethyl-3-(3-dimethylaminopropyl)-carbodiimide (EDC; 488 mg, 3.15 mmol) is added in ice chilling. Two hours later, 1-hydroxybenzotriazole (HOBt; 193 mg, 1.43 mmol), Aoc-Arg(Tos)-OH • 1/4 AcOEt 1/2 $H_2O$ (675 mg, 1.43 mmol), and EDC (224 mg, 1.44 mmol) are added. The mixture is allowed to react for 3 hours at 4°C. DMF is distilled away under reduced pressure, the residue is dissolved in chloroform, washed successively with cold 1M-hydrochloric acid and saturated sodium bicarbonate solution, and dried on magnesium sulfate. The filtrate is concentrated and the residue is refined by silica gel column chromatography (120 g, 97% chloroform-3% methanol) and precipitated from methanol-ethyl acetate to yield 2.14 g of the captioned compound (yield 54%); mp 175 -178°C.

4. Preparation of Boc-Cys(Acm)-Phe-Gly-Gly-Arg(Tos)-Met-Asp(OBzl)-Arg(Tos)-Ile-Gly-OH (VI:$R^1$ = Acm; $R^3$ = Boc)

(1) Boc-Cys(Acm)-Phe-Gly-Gly-OBzl ( 31)

Compound 24 (943 mg, 2.01 mmol) is treated with TFA (5 ml) for 30 minutes in ice chilling. TFA is distilled away under reduced pressure and the residue is dissolved in chloroform, washed with saturated sodium bicarbonate solution, and dried on magnesium sulfate. The filtrate is evaporated under reduced pressure and the residue is dissolved in DMF (10 ml), and Boc-Cys(Acm)-OSu (935 mg, 2.4 mmol) is added. The mixture is allowed to react for 18 hours at 23 °C. The oil obtained by distilling away the DMF is dissolved in ethyl acetate, washed with water, and dried on magnesium sulfate. The filtrate is evaporated under reduced pressure and the residue is recrystallized from methanol-ethyl acetate-petroleum ether to yield 1.18 g of the captioned compound (yield 92%); mp 143 - 144°C.

(2) Boc-Cys(Acm)-Phe-Gly-Gly-$N_2H_3$ (32)

Compound 31 (1.09 g, 1.69 mmol) is dissolved in methanol (10 ml) and hydrazine hydrate (0.41 ml, 8.44 mmol) is added. The mixture is allowed to react for 18 hours at 23°C. The solvent is distilled away under reduced pressure and the residue is evaporated to dryness under reduced pressure. The resulting residue is recrystallized from the methanol-ethylether to yield 933 mg of the captioned compound (yield 97 %); mp 172 -175 °C.

(3) Boc-Cys(Acm)-Phe-Gly-Gly-Arg(Tos)-Met-Asp(OBzl)-Arg(Tos)-Ile-Gly-OTce (33)

Compound 32 (394 mg, 0.66 mmol) is dissolved in DMF (5 ml), and 4M hydrochloric acid-dioxane (0.30 ml, 1.2 mmol) and isoamyl nitrite (0.12 ml, 0.9 mmol) are added at -20°C. Further cooling to -60°C in 20 minutes, tri-n-butylamine (0.6 ml, 2.5 mmol) and DMF (2.5 ml) solution of H-Arg(Tos)-Met-Asp(OBzl)-Arg(Tos)-Ile-Gly-OTce • TFA salt prepared by treating compound 30 (834 mg, 0.6 mmol) in TFA are added. The mixture is allowed to react for 18 hours at 4°C. DMF is distilled away under reduced pressure and ethyl acetate is added to the residue. The precipitate is reprecipitated from DMF-methanol to yield 986 mg of the captioned compound (yield 91%); mp 222 -224°C (decomposed).
$[\alpha]_D$ -15.9 ± 0.6° (23 °C, c 1.0, DMF).

(4) Boc-Cys(Acm)-Phe-Gly-Gly-Arg(Tos)-Met-Asp(OBzl)-Arg(Tos)-Ile-Gly-OH (VI)

Compound 33 (936 mg, 0.516 mmol) is dissolved in dimethyl formamide (DMF) (6 ml) and acetic acid (2 ml) is added. Zinc powder (720 mg, 11 mmol) is added thereto and the mixture is stirred for 30 minutes at 23°C. The insoluble material is filtrated away and the filtrate is evaporated under reduced pressure. The resulting residue is washed with 1M hydrochloric acid and precipitated from DMF-methanol to yield 846 mg of the caption compound (VI) (yield 98 %); mp 205 - 208 °C.

It is confirmed as showed in the following experiment the β-hANP (7-28) (I) prepared by this invention (hereinafter called peptide I) has a diuretic action to animals and that the peptide can be used as a diuretic hypotensive agent.

Experiment

Transition of urine volume and excreted sodium ion volume

[Method]

Male Slc-SD rats (weighing about 350 g) were anesthetized by intraperitoneal administration of 30 mg/kg of sodium pentobarbital, and cannulae were placed in the femoral part and the urinary bladder, and physiological saline was injected from the vein at a rate of 0.15 ml/min. The bladder cannula was led into a drop counter to collect urine, and the urine volume and sodium ion volume ($Na^+$) in urine per unit time were measured. The test material (α-hANP, peptide I) was dissolved in physiological saline, and was injected from the venous cannula at a rate of 0.1 ml (1 µg or 3 µg)animal.

[Results]

By administration of α-hANP 1 µg/animal, both urine volume and $Na^+$ excretion volume increased immediately right after administration, and reached the peak in 1 to 2 minutes, and dropped rapidly to reach the normal value in 10 minutes. On the other hand, by 3 µg/animal administration of peptide I, the increase of urine volume and $Na^+$ excretion was mild, and the peak values were lower than those by α-hANP administration, and the decrease was also slow, and higher values persisted for 20 to 30 minutes after administration. The results are shown in Fig. 1 (transition of urine volume) and Fig. 2 (transition of $Na^+$ excretion volume).

Peptide I of this invention is useful as a hypotensive diuretic, and it may be used, for example, in the prevention or treatment of essential hypertension, cardiac edema (congestive heart failure) and malignant hypertension. Peptide I may be usually administered nonorally, for example, intravenously, intradermally, subcutaneously, intramuscularly, nasally or intrarectally, and as the form of dosage, solution for injection, suspension or drip infusion may be applicable. The dose is determined depending on the age, body weight and symptons of patients, and usually the daily dose is about 500 ng/kg to 5000 ng/kg, or preferably 750 ng/kg to 2000 ng/kg, which may be administered either once or by several divided portions a day.

The peptide I of this invention is also useful as an intermediate form of synthesis of β-hANP or its analogous compounds.

## Claims

A polypeptide represented by the general formula:

General formula:

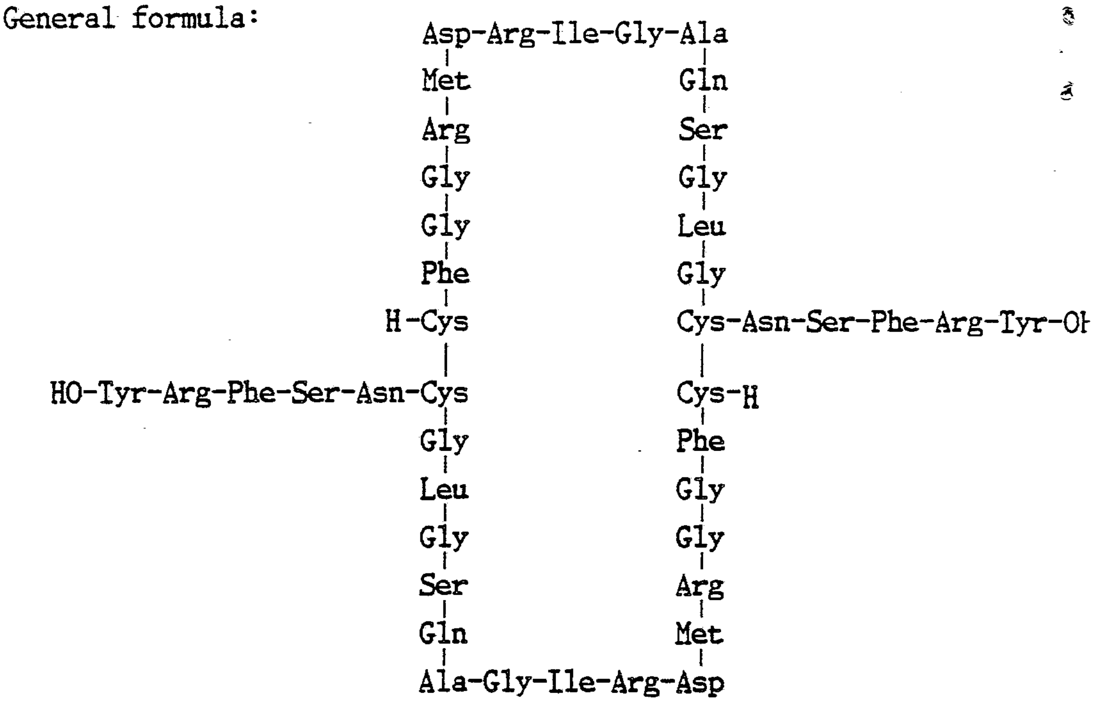

and the salts thereof.

Fig. 1

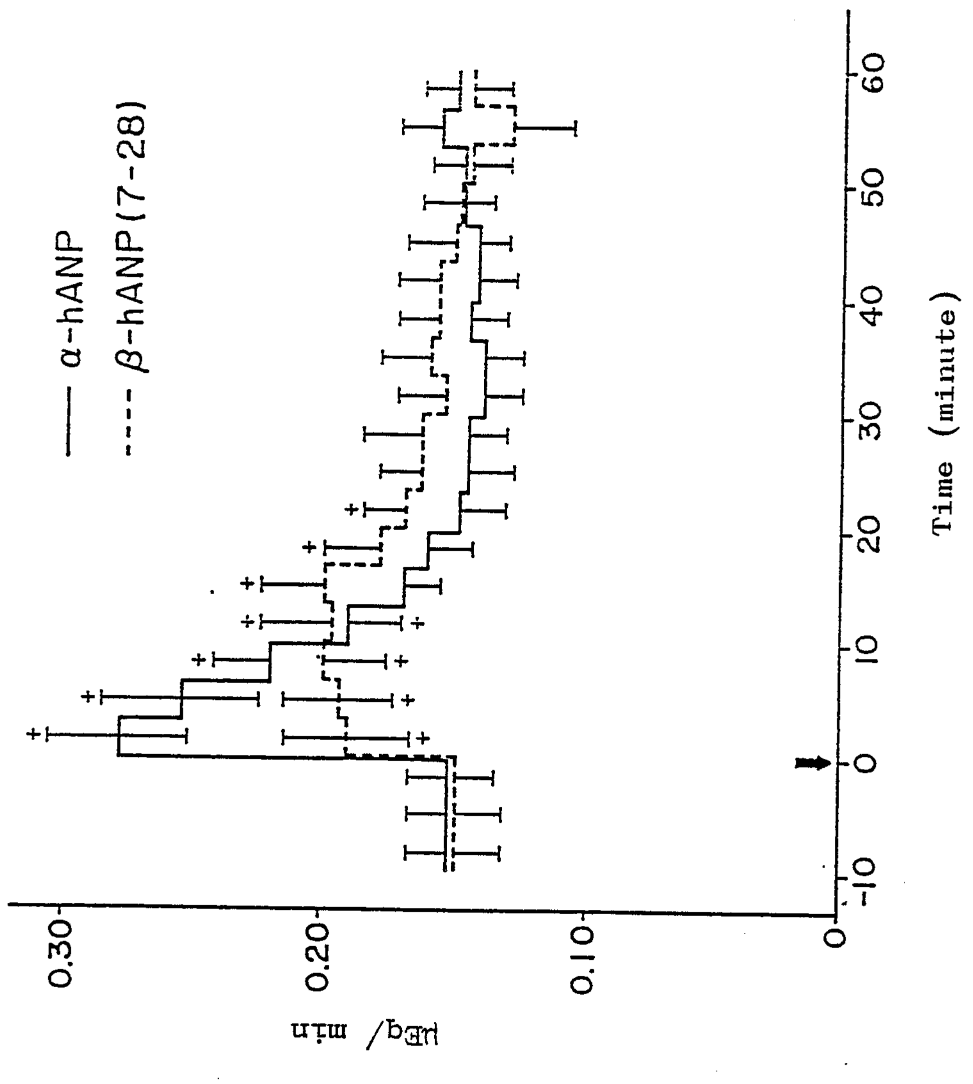
α-hANP
β-hANP(7-28)
0.30
0.20
0.10
0
μEq/ min
-10
0
10
20
30
40
50
60
Time (minute)

Fig. 2

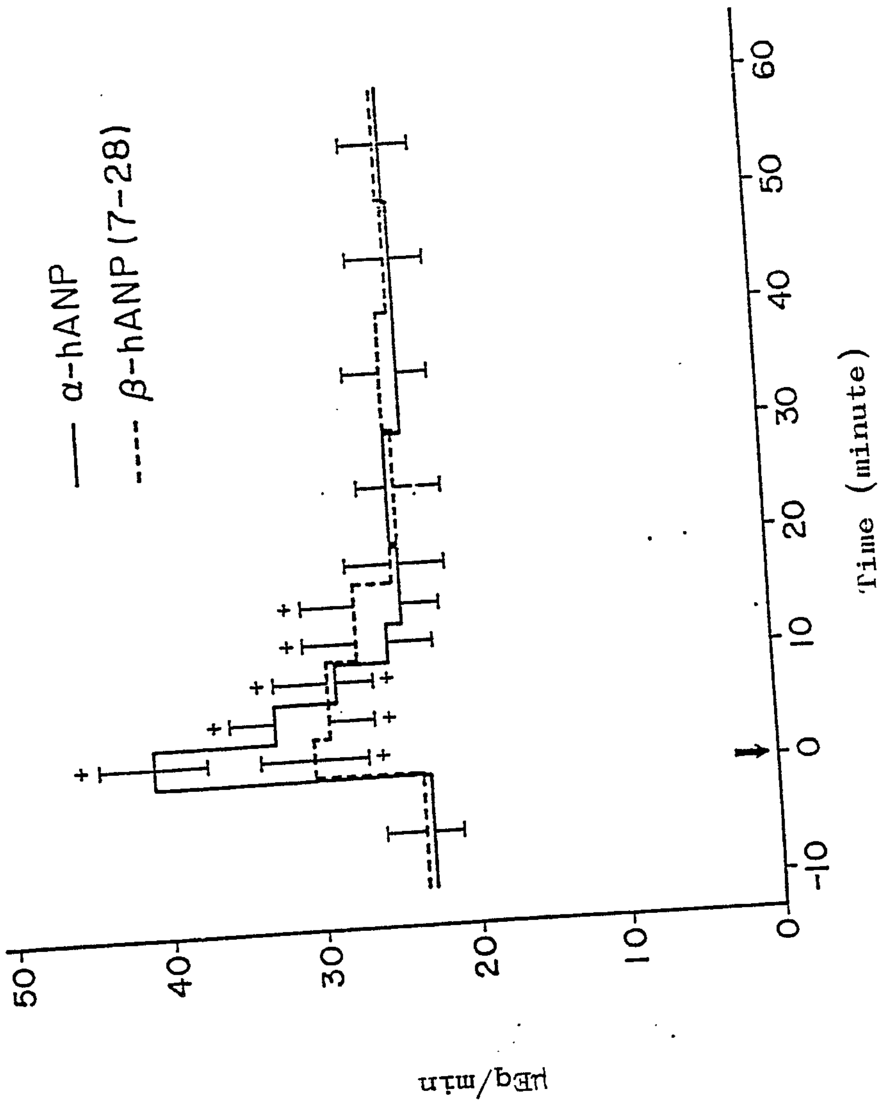
α-hANP
β-hANP (7-28)
μEq/min
0
10
20
30
40
50
-10
0
10
20
30
40
50
60
Time (minute)